# EUROPEAN PATENT APPLICATION

(11) **EP 2 386 327 A1**
(43) Date of publication of application: **16.11.2011**
(21) Application number: 10162692.7
(22) Date of filing: 12.05.2010
(51) Int. Cl.: A61M 39/08, B65H 75/40, H02G 11/02, A61M 5/14

(54) **Device for adjusting the length of infusion tubing**

(71) Applicant: F. Hoffmann-La Roche AG, 4070 Basel (CH); Roche Diagnostics GmbH, 68305 Mannheim (DE)
(72) Inventor: Teutsch, David, 3251, Wengi (CH)
(74) Representative: Rentsch Partner AG

(57) **Abstract**

A device (1) for storing and dispensing infusion tubing (2) comprises a casing (6, 81), a spool (3, 84) for storing the tubing (2), rotatably mounted (35, 64) on the casing (6), and a locking structure (31, 311, 312) for positively locking a central portion (21) of a tubing (2) on the spool (3, 84). A ratchet-like locking mechanism (5, 52, 81, 82) locks a rotation of the spool (3) in regard to the casing (6) in at least one direction (A, B).

## Description

### Field of the Invention

The invention relates to devices for adjusting the length of infusion tubing, with a casing and a spool for winding up the tubing, rotatably mounted on the casing.

### State of the art

Infusion pump devices are a modern and convenient way to deliver liquid medicaments to patients by injection or infusion. Particularly useful are infusion pump devices in diabetes therapy and pain therapy, where patients need small doses of their respective medication on a regular basis. Modern infusion devices as sufficiently small to be carried concealed on the body, which is much appreciated by patients that are otherwise healthy and follow their normal daily activities.

Depending on the particular type of infusion pump device used, flexible infusion tubing is used to fluidly connect the infusion pump device, located on a convenient place on the body of the patient, with the infusion site, where the liquid medicament is conveyed into the body of the patient through a needle or cannula penetrating the skin.

The shortest distance between the pump device and the infusion site defines the minimum length of the infusion tubing. However, infusion tubing is often available only in standardized length units, e.g. 1 meter. Excess tubing cannot simply be removed by the user. On one hand, cutting the tubing compromises sterility. On the other hand typically connectors are attached at the ends of the tubing for fluidly connecting the tubing to other devices. Furthermore a user may need to change the position of the infusion pump device during the course of the day. He may for example wish to place the infusion pump on the bedside table during sleep.

To avoid the necessity to exchange the tubing every time, the user will choose a tubing that is sufficiently long for all needs during the normal life time of an infusion tubing. This also avoids the loss of expensive medication remaining in the discarded tubing. As a consequence there is in most cases surplus infusion tubing that has to be handled with by the user. For example may the user gather the surplus tubing and fixate it in coils with adhesive tape, e.g. on the pump device or on the body. However, depending on the amount, the tubing may be difficult to conceal. Uncoiling the infusion tubing can be also a difficult and time-consuming task, depending on the adhesive tape used. Furthermore there is a permanent risk of the infusion tubing becoming tangled or kinked or caught on an obstacle.

US 2003/0122021 A1 discloses a device for adjusting the length of infusion tubing, with a spool with a central hub. The hub comprises a passage through which the infusion tubing is threaded. The tubing is then wound up on the spool, thereby retracting the tube on both sides onto the spool and adjusting the overall length of the tube.

US 2006/0065772 A1 teaches a device in which a hub with a flat base and with three posts that are rotatably arranged on a cylindrical casing. The tubing enters and leaves the casing through opposite openings on the rim of the casing and is threaded through the posts. Rotating the casing in regard to the hub retracts the tubing. The device is difficult to disassemble, since the three posts engage behind a central opening of the cover. The user has to bend back all three posts to remove the cover from the hub, which requires a considerable manual aptness. A similar device is disclosed in WO 02/46080 A1.

The devices known from the prior art have the disadvantage that the retraction and release of the tubing cannot be precisely controlled.

### Objects of the Invention

It is an object of this invention to provide a device for adjusting the length of infusion tubing that overcomes the above-mentioned and other problem. Particularly such a device should allow a user to easily adjust the length of infusion tubing and to store surplus infusion tubing. The tubing should not be damaged, kinked or otherwise compromised when stored in the device.

Another object of the invention is to provide a device for adjusting the length of infusion tubing that can be easily coupled to existing and already mounted infusion tubing, without the need of disconnecting the tubing.

A device according to the inventions should be producible at low costs, and should be reliable and easy to use.

These and other objects are achieved by a device, a set and an infusion tubing according to the independent claims. Advantageous embodiments are given in the dependent claims.

### Summary of the invention

A device according to the invention for storing and dispensing infusion tubing comprises a casing, a spool for storing the tubing, rotatably mounted on the casing, and a locking structure for positively locking a central portion of a tubing on the spool. The device comprises a ratchet-like locking mechanism suitable for locking a rotation of the spool in regard to the casing in one or both directions.

In the context of this specification, the term □ locking □ does include both absolute locking, in which a rotation of the spool in regard to the casing is mechanically prevented, as well as a relative locking, which allows rotation of the spool after exceeding a threshold force for releasing the ratchet-like mechanism. Advantageously, in an embodiment of a device according to the invention with absolute locking, such absolute locking applies only in one direction, while in the other direction relative locking or no locking may apply.

In another advantageous embodiment, a device according to the invention comprises guiding structures for the tubing.

In a particularly advantageous embodiment of a device according to the invention, the locking mechanism is a ratchet mechanism comprising a ratchet gear consisting of a multitude of circularly arranged grooves or teeth, and one or more paws interacting with the ratchet gear. The ratchet gear is arranged on an outer delimiting flange of the spool, and the paws are attached to the casing, or vice versa.

In another particularly advantageous embodiment of a device according to the invention the spool comprises a bore rotatably mounted on a hub attached to the casing. The locking mechanism is a ratchet mechanism comprising a ratchet gear consisting of a multitude of circularly arranged grooves or teeth, and one or more paws interacting with the ratchet gear. The ratchet gear is arranged on the circumference of the bore, and the paws are arranged on the hub, or vice versa.

A device according to the invention may comprise an actuation mechanism, particularly a spring coil, for rotating the spool in one direction.

In another useful variant of the device according to the invention, an outer delimiting flange of the spool comprises two radial slots on opposite sides of the flange, extending to axial slots in the spool body. Advantageously the device comprises guiding structures for the tubing, with openings through which the tubing can run to the spool. Said openings comprise slots facing toward the same direction as the slots on the flange of the spool.

In yet another embodiment of a device according to the invention, the spool is realized as an inner unit in the form of a cylinder, having an outer thread and two or more areas without threading. The casing is realized as an outer unit in the form of a cylinder barrel. In an advantageous variant of such an embodiment the cylinder barrel like outer unit is provided with an inner thread and two or more areas without threading, or other means for guiding the tubing, such as grooves or slots.

The dimensions of the inner and outer unit and the threads are chosen in such a way that tubing with a certain diameter can be wound up to the inner unit, forming a tubing coil. If there is an inner thread of the outer unit, said tubing coil can be screwed into the thread of the outer unit.

A device according to the invention can be provided with a tubing wound up to the spool. Advantageously the device including the tubing is sterilized. The tubing can comprises connectors on one or both ends for connecting the tubing to an infusion pump device and/or an infusion cannula or needle.

A device according to the invention may be fastened to the body by adhesive tape, or to the belt or the clothes by using a suitable clip or hook-and-loop fastener (Velcro®). It may also be realized as an integral part of a pump casing.

Advantageously a device according to the invention is produced by injection moulding, using suitable polymer materials. The dimensions of the device are mainly given by the length and diameter of the tubing to be stored.

A set according to the invention comprises a device according to the invention as discussed above, and one or more pieces of tubing.

An infusion tubing according to the invention comprises one or more devices according to the invention as discussed above that is attached to the infusion tubing.

### Brief description of the drawings

In order to facilitate a fuller understanding of the present invention, reference is now made to the appended drawings. These references should not be construed as limiting the present invention, but are intended to be exemplary only.
- Figure 1: shows an embodiment of a device according to the invention with automatic retraction functionality.
- Figure 2: shows another of a device according to the invention with automatic retraction functionality.
- Figure 3: shows a detail of the ratchet mechanism of the device in Figure 2.
- Figure 4: shows a device according to the invention that is manually operated.
- Figure 5: shows another device according to the invention that is manually operated, and in which the tubing can be inserted without interrupting the fluid connection.
- Figure 6: shows a further embodiment of a device according to the invention.
- Figure 7: shows the application of the device shown in Figure 6.

### Description of embodiments of the invention

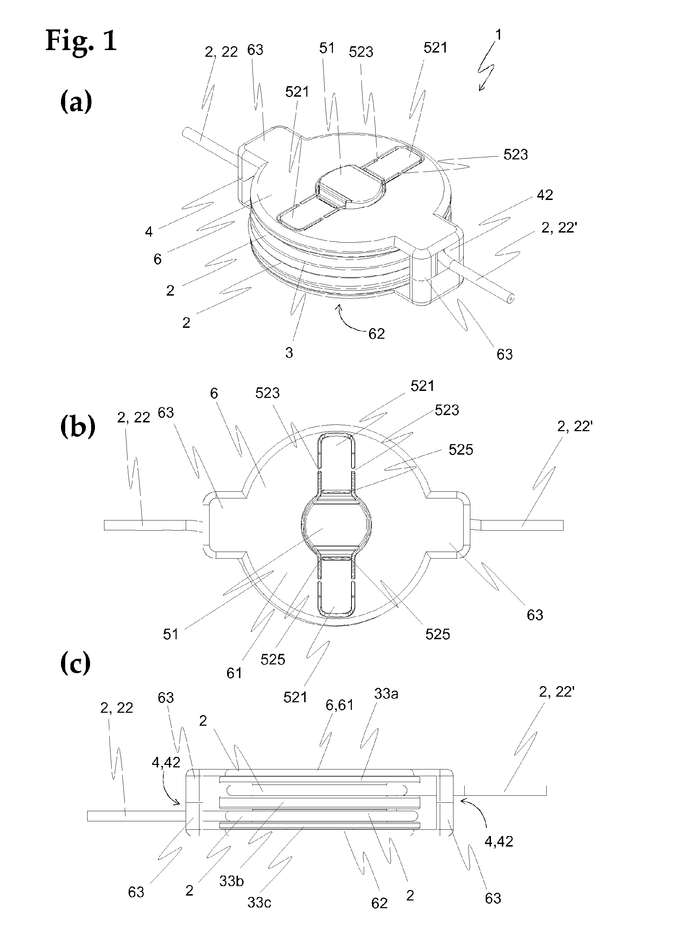

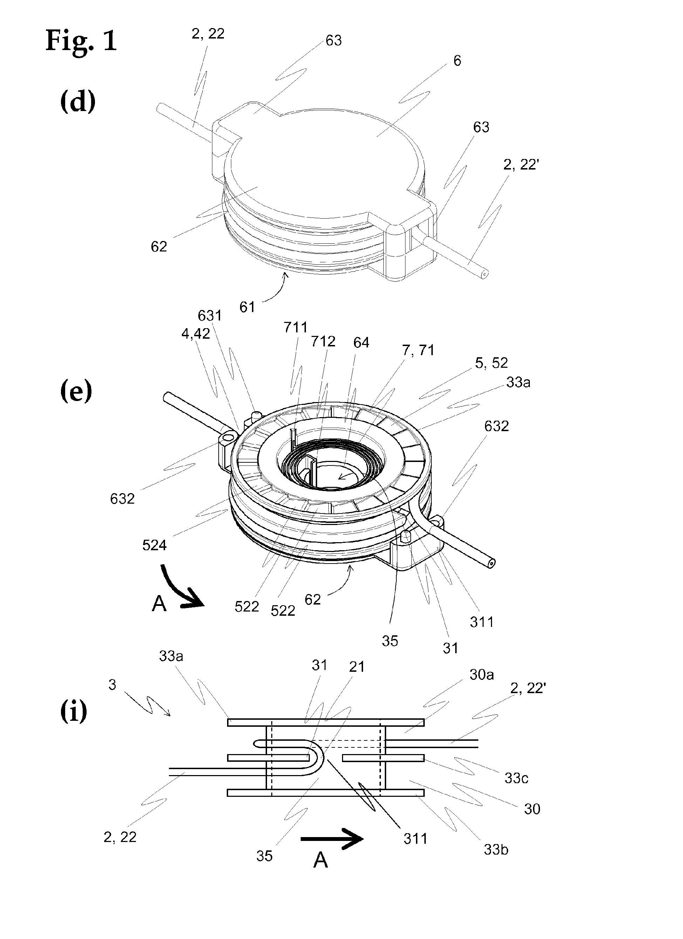

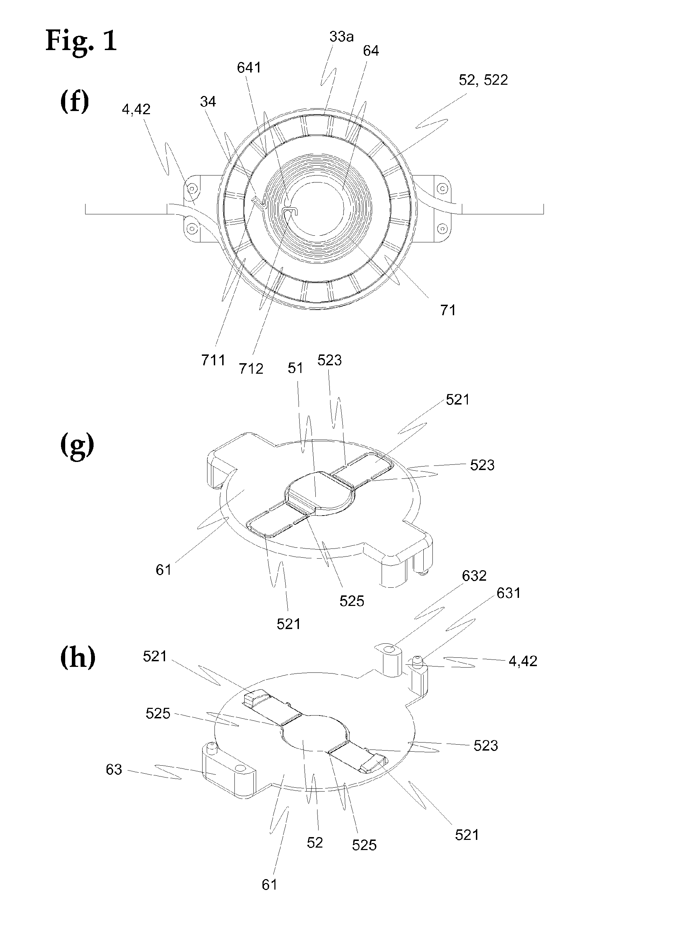

A first advantageous embodiment of a device 1 according to the invention is shown in Figure 1, (a) in a perspective view toward the top cover 61, (b) in a top view, (c) in a side view, (d) in a perspective view toward the bottom cover 62, and (e), (f) with removed top cover 61. Figures 1 (g) and (h) show the removed top cover 61 from both sides. Figure 1 (i) schematically shows a side view of the spool 3, with visible tube locking structure 31.

The embodiment of the device shown in Figure 1 comprises a spool 3 that is rotatably mounted in a casing 6, comprising a top cover 61 and a bottom cover 62. The top cover 61 and the bottom cover 62 are connected via two bridge structures 63. During use the infusion tubing 2 is wound onto the spool 3. Figure 1 shows the device with the tubing 2 already wound up. The tubing leaves the casing via two guiding structures 4, realized as two openings 42 in the bridge structures 63. Said guiding structures 4 direct the tubing toward the spool, and prevent the tubing from unwinding itself when the spool is locked in its rotational movement.

The device 1 is particularly advantageous for use in a prepacked set, with a device provided with the infusion tubing 2 already wound up, in a sterile package. After unpacking the device, the user will hold the two ends 22, 22' of the tubing, sticking out of the device, and will pull both ends into opposite directions. The tubing will unwind from the device, until the necessary amount of tubing is released. The remaining central portion of the tubing remains coiled on the device. One end 22 of the infusion tubing is then connected with a suitable connector (not shown) to an infusion pump device (not shown) or a similar medical device, or even to a standard IV system. Similarly the other end of the tubing is connected to an infusion cannula (not shown), which has been inserted into the patient □ s body prior tonnecting it to an infusion tubing. In other embodiments, the infusion cannula is realized as an integral part of the tubing, in which case the infusion cannula is inserted into the patient □ s body after uinding the necessary amount of tubing from the device.

The embodiment of the device depicted in Figure 1 comprises automatic retraction functionality. By pressing the release button 51 arranged in the centre of the top cover 61, two paws 521 of a ratchet mechanism 52 are temporarily removed from the teeth 522 of a ratchet gear, thereby unlocking the ratchet mechanism 52. A biased spring coil 71 actuates and rotates the spool 3, rewinding the tubing 2 onto the spool 3. When the button 51 is released, the biased paws 521 hook into the teeth 522, and the ratchet mechanism 52 is locked again. When a user pulls both ends 22, 22' of the infusion tubing 2, the inclined planes of the paws and teeth glide on each other, according to the standard principle of a ratchet mechanism. A user thus may very easily adjust the length of the tubing. He can pull the ends of the tubing to release the tubing from the device, and can retract surplus tubing into the device 1 by pressing the button.

The ratchet mechanism 52 is shown in more detail in Figures 1(e) to (h). The exterior side of one delimiting flange 33a of the spool is provided with a circumferential ratchet gear 524 with teeth 522. The spool comprises a central bore 35, which is rotatably mounted on a central, hollow hub 64 protruding from the inner side of the bottom cover 62. A spring coil 71 is arranged in the bore 35 of the spool 3, its first end 711 being positively locked in a slot 34 of the spool, and the second end 712 being positively locked in a slot 641 of the hub 64. The teeth 522 of the ratchet gear 524 interact with two paws 521. Said paws are pivot-mounted on hinge areas 523 and connected to the release button 51 via hinge areas 525. Thus paws 521 and release button 51 are connected with the top cover 62 only via the four hinge areas 523.

When the release button 51 is pressed inwards, the paws 521 are pivoted away from the teeth 522, thereby releasing the ratchet mechanism 52. The deformation of hinge areas 523, 525 results in a bias, and upon release of the button 51 the paws are forced back toward the teeth, relocking the ratchet mechanism. The top cover with paws and button can be manufactured as a single piece by injection moulding. The hinge areas 525 can for example be realized as film hinges between paws and button. In the embodiment shown in Figure 1, the hinge areas 523, 525 are realized as narrow rips.

Preferably the spring coil 71 is biased in such a way that the spring force is sufficiently strong to rewind the tubing, but also sufficiently weak to be overcome by a user without damaging the tubing. Furthermore the retracting speed should preferably be low, to give the user precise control over the retraction.

In a device according to the invention, both ends 22, 22' of the tubing are retracted at the same time, or released at the same time. The device uses a single spool, thereby avoiding a complicated gear mechanism and reducing the overall number of parts. As a consequence the two ends of the tubing 22, 22' have to be wound up on the spool in the same direction. For this purpose a structure 31 is provided on the spool that allows to positively lock a central portion 21 of the tubing to the spool. When the spool is rotated, both ends are then wound up in the same direction.

In the embodiment of a device according to the invention as shown in Figure 1, the spool 3 is subdivided into two separate spool compartments 30, 30a, by two outer delimiting flanges 33a, 33b and a central delimiting flange 33c. This is schematically shown in more detail in Figure 1(i). Each end 22, 22' of the tubing is wound onto one of said spool compartments 30, 30a. The use of two separate compartments has the advantage that the two ends of the tubing cannot mingle with each other during the winding and unwinding procedure.

The central delimiting flange is provided with one or more radial slots 311, having a width that is broader than the diameter of the tubing to be stored. In a maximum unwound state of the device, the tubing 2 will run through a first opening 42 of the guiding structure 4, and along a first spool compartment 30a. It will then pass one of the slots 311 in the central flange 33c into the second compartment 30a, where it reverses its direction, runs along the second spool compartment 30a, and through a second opening 42 out of the casing. When the spool is now rotated in one direction of rotation A (shown as a large arrow), the edge 31 of the central flange 33c neighbouring the slot 311 will drag both ends of the tubing in this direction, and the tubing is retracted into the device.

In a particularly advantageous embodiment of such a device, the casing further comprises a circumferential wall. In such a device the spool is fully enclosed in the casing.

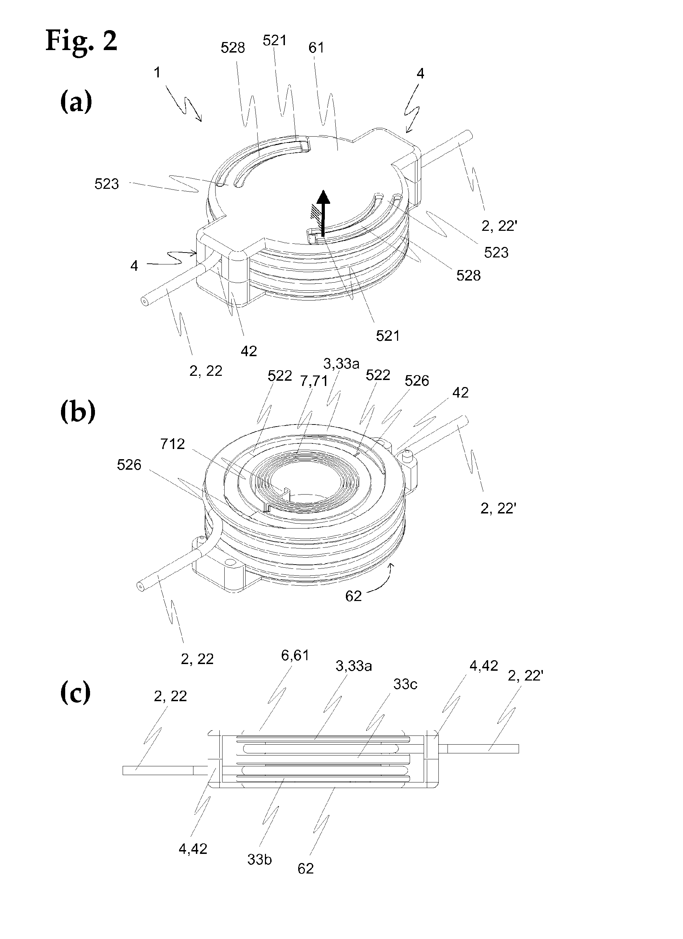

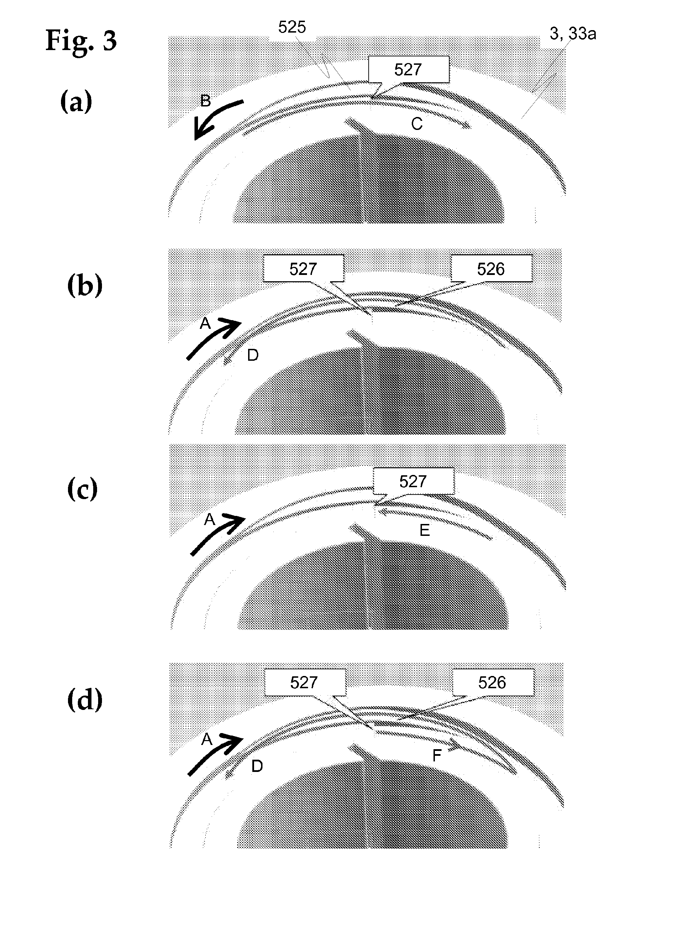

Figure 2 shows another embodiment of a device 1 according to the invention, also with automatic retraction, (a) in a perspective view, (b) in a perspective view with removed top cover 61, and (c) in a side view. The shown embodiment differs from the device in Figure 1 essentially by its locking mechanism. While in Figure 1 a ratchet mechanism 52 controlled by a button is used to stop the retracting motion of the spool driven by the spring coil, the embodiment in Figure 2 uses a self-locking and unlocking ratchet mechanism 52, shown in more detail in Figure 3.

The spool 3 comprises a ratchet gear with four teeth 522, arranged on one of the outer delimiting flanges 33a. Two paws 521 are formed as an integral part of the top cover 61. The paws are connected to the cover 61 by long necks 528, the connection between the neck and the cover acting as a hinge area 523.

When a user pulls the ends 22, 22' of the tubing 2 into opposite directions, the spool 3 rotates in the releasing direction B, and the paws move along the path C on the ratchet gear, by jumping over the barriers 527 of the teeth 522 (see Figure 3(a)). During this movement each paw is bent out of the plane by the flat ramp of tooth 522, and is also bent radially inwards by the barrier of a second, reversed ramp 526. The user thus can increase the length of the tubing by pulling it out of the device. When the user stops pulling the tubing 2, the spool will start retract the tubing, actuated by the biased spring coil 71. The paws will follow a path D over the reverse ramp 526 and jump over the barrier of the reverse ramp (see Figure 3(b)).

The user can then stop the tubing from retracting, and can pull it out far enough for letting the paws jump over the barrier 527 of teeth 522, but not far enough for letting them reach the begin of the reverse ramp 526. When the user releases the tubing again, the spool will be rotated by the coil 71 in the retraction direction A. However, since the paws are not on the reverse ramps 526, each paw is bent radially inwards by the barrier of the ramp 526 and returns E to the barrier 527 of the tooth 522, where paws 521 and teeth 522 pairwise hook into each other and lock the ratchet mechanism (see Figure 3(c)).

To unlock the ratchet mechanism, the user pulls out the tubing far enough that the paw reaches F the begin of the reverse ramp 526 (see Figure 3(d)). When now the user releases the tubing again, the spool can again rotate unhindered in the retraction direction A, while the paws follow path D along reverse ramp 526.

An embodiment of a device according to the invention as it is disclosed in Figures 2 and 3 has the advantage that no button has to be pressed by a user, which may be difficult when the device is placed on a flexible underground, for example on the abdomen of a user. The user may release and retract the tubing according to his wishes, and lock and unlock the retraction function, just by simply increasing and decreasing the pulling force on the tubing.

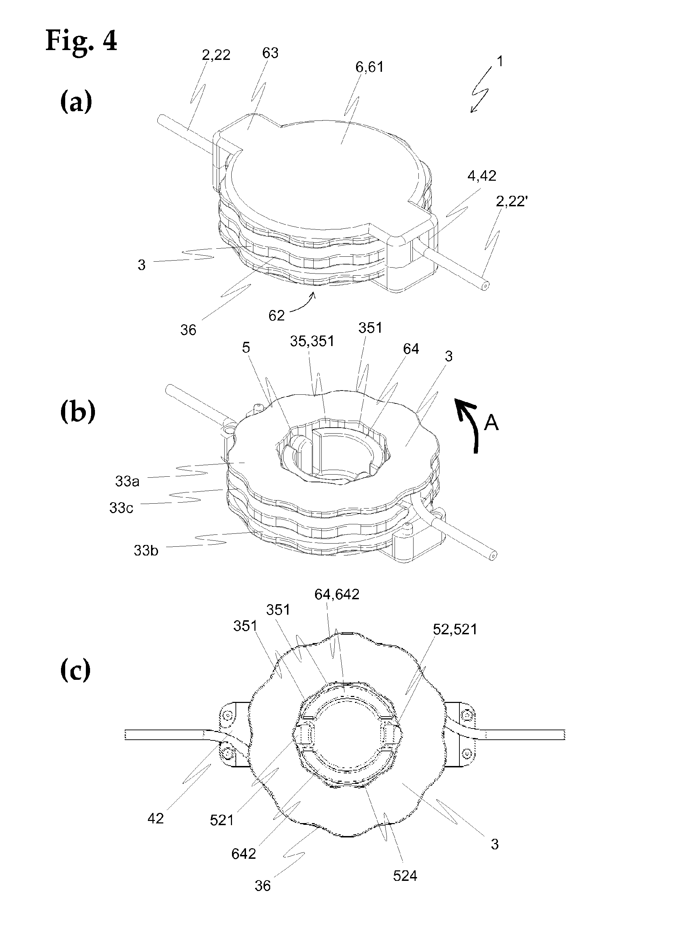

Again, the casing of the device of Figure 2 may comprise a circumferential wall, protecting the content of the spool from mechanical damage. Both embodiments in Figure 1 and 2 may also be realized with a single spool compartment, as in the embodiment shown in Figure 3 and 4.

In a variant of the disclosed device according of the invention with ratchet-like locking mechanism, said ratchet mechanism can be realized as part of the bore of the spool and the hub on which the spool is mounted. Similarly the spring coil alternatively can be arranged between an outer delimit flange and a cover the casing.

Other advantageous embodiments of a device according to the invention can be realized fully hand operated, without the need for a retracting spring coil or a similar actuation mechanism. An example of such an embodiment is depicted in Figure 4, (a) in a perspective view, (b) in a perspective view with removed top cover 61, and (c) in a top view with removed top cover.

The device comprises a casing 6 with top cover 61 and bottom cover 62, in which the spool 3 is rotatably mounted on a hub 64 protruding from the inner side of the bottom cover 62. The spool 3 is realized with two compartments, similar to Figures 1 and 2, and also the guiding structures 4 are the same. To release the tubing from the device, the user pulls the ends 22, 22' of the tubing. To retract the tubing into the device, the user manually turns the spool 3 along the retraction direction A. For that purpose, one or more of the outer edges of the delimiting flanges 33a, 33b, 33c are provided with suitable gripping means 36. In the given example the edges are provided with depressions for better grip. Other possibilities are for example a knurling on the edges of the spool, or a coating with a suitable material with increased grip, for example a soft, rubber-like polymer.

As in the previously discussed embodiments, the device according to the invention is provided with a ratchet-like 52 locking mechanism 5. For that purpose the hub 64 is subdivided into two guiding components 642, both having essentially the form of a half circle, and two paws 521 protruding perpendicularly from the bottom cover 62 and being arranged between the guiding structures 642. The essentially cylindrical bore 35 of the spool has a corrugated surface, with a number of axially oriented grooves 351. In the given example there are ten grooves. The outer diameter of the hub guiding structures 642 corresponds to the minimum diameter of the bore 35, between the grooves. The outer distance of the paws 521, on the other hand, corresponds to the maximum diameter of the bore measured between two opposite grooves.

If a user pulls the tubing, thereby rotating the spool in the release direction B, or manually turns the spool in the retraction direction A, the two paws 521 of the ratchet mechanism are bent inwards by the convex area between the grooves 351 of the bore, and engage again with the neighbouring grooves 351. This deformation of the paw 521 requires a certain force. Thus the spool will immediately stop its rotation in regard to the casing when the tubing or the spool is released by the user, and the tubing cannot accidentally unwind itself.

A particular advantage of the embodiment of a device as shown in Figure 4 is that the ratchet mechanism unlocks itself if the pulling force on the tubing exceeds a certain threshold. If a user accidentally hooks the tubing in an obstacle when moving, for example a door knob, the ratchet mechanism will unlock, and tubing will be released. This prevents the infusion needle to be ripped of out of the body of the user, which is rather painful. Such a device according to the invention thus also acts as a security measure to prevent injuries of the user. Furthermore the disclosed ratchet-type mechanism allows a user to precisely control the retraction and release of the tubing, which is a great advantage, compared for example to simple frictional locking of the spool on the hub.

In an alternative embodiment of such a device, the paws can be arranged in the wall of the bore, and the hub can be provided with grooves.

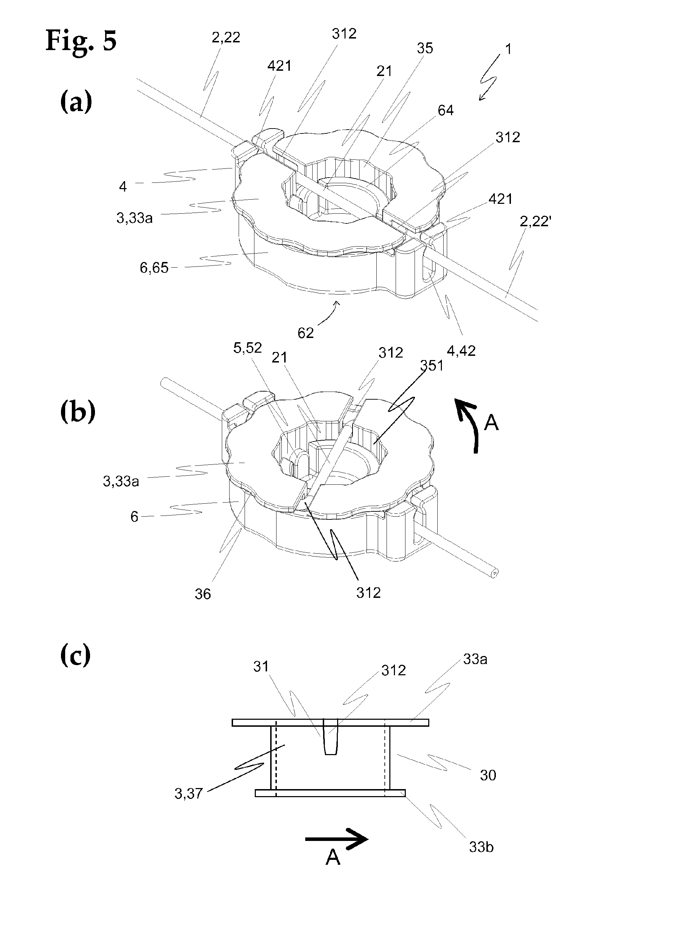

Another manually operated embodiment of a device 1 according to the invention is depicted in Figure 5. In this particularly advantageous embodiment, the tubing can be inserted without disconnecting its ends, for example from the infusion cannula or the infusion pump.

Figure 5 shows (a) a perspective view of the device 1 directly after threading the tubing 2 into the device, (b) a perspective view of the device after a rotation of the spool 3 of about 105° along the retraction direction A, and (c) a side view of the spool.

Said device comprises a casing 6 and a spool 3. The casing comprises a bottom cover 62, a circumferential wall 65 and two guiding structures 4, protruding from the wall 65. The spool comprises two outer delimiting flanges 33a, 33b, defining one single tubing compartment. The spool 3 is rotatably mounted on the casing 6, with the bore 35 of the spool being arranged on the hub 64 attached to the inner side of the bottom cover 62, similar to the embodiment in Figure 4. A ratchet mechanism 52 similar to Figure 4 is used as the locking mechanism 5 of the device 1. To allow the manual rotation of the spool by the user, the upper flange 33a extends over the wall 65 and is provided with a grip structure 36.

Since the casing 6 does not comprise a top cover 61, the spool 3 is not positively locked between two covers 61, 62. Thus the spool not only has to be locked in its rotational movement on the hub, but also its position on the hub axis has to be secured. For that purpose the grooves 351 on the wall of the bore extend in the axial direction only over a central part of the bore. When the spool is placed on the hub, the paws of the ratchet mechanism snap into the grooves, thereby locking the spool on the hub in the axial direction.

To allow the threading of the fluidly connected tubing into the device 1, the openings 42 of the guiding structures 4 are provided with slots 421 toward the upper side of the device. Two opposite slots 312 extend over the upper flange 33a of the spool and an upper portion of the spool body 37. The width of the slots 312 is equal or wider than the diameter of the tubing. The width of the slots 421 of the openings 42 are preferably slightly smaller than the tubing 2, to prevent the tubing 2 from leaving the opening 42. To insert the tubing 2 into the device 1, the slots 312 of the spool 3 and the slots 421 of the guiding structure 4 are aligned. The user now can insert the tubing 2 into the slots 312, 421, as shown in Figure 5(a). Since the slots of the guiding structure are narrower than the tubing, it is temporarily compressed during insertion. The tubing now runs through the openings 42 of the guiding structures and the slots 312 of the spool body 37.

With the tubing 2 inserted, in a next step the user will rotate the spool 3 in a retracting direction A. The edges 31 of the slots 312 will drag the tubing 2 along the direction of rotation, and both ends 22, 22' of the tubing are retracted into the device and winded up to the spool compartment 30.

Since the device of Figure 5 can be attached and removed from the tubing at any time without disconnecting the tubing, it can be used in a very flexible manner. During the night a user may place the infusion pump device on his bedside table. The pump is fluidly connected to the infusion cannula by a tubing 2. The length of the tubing is sufficient to take into account any movements of the user during sleep. After getting up the user will attach the pump device to his body or his clothes. He then threads the tubing into the device according to the invention, or mounts the device to the tubing, respectively. He then turns the spool to retract the surplus tubing as far as necessary, and attaches the device to his body or his clothes.

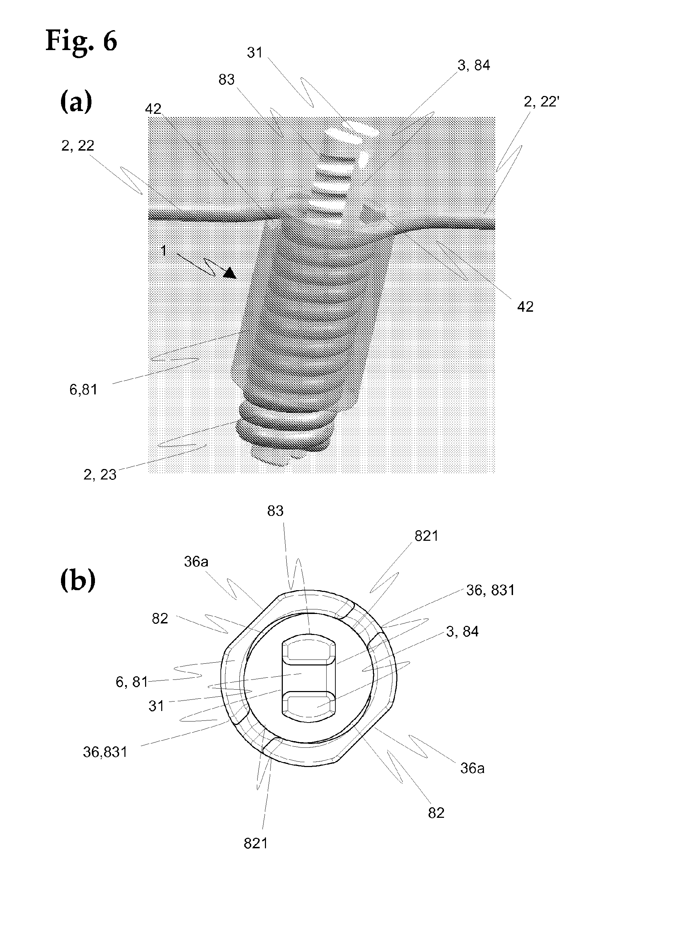

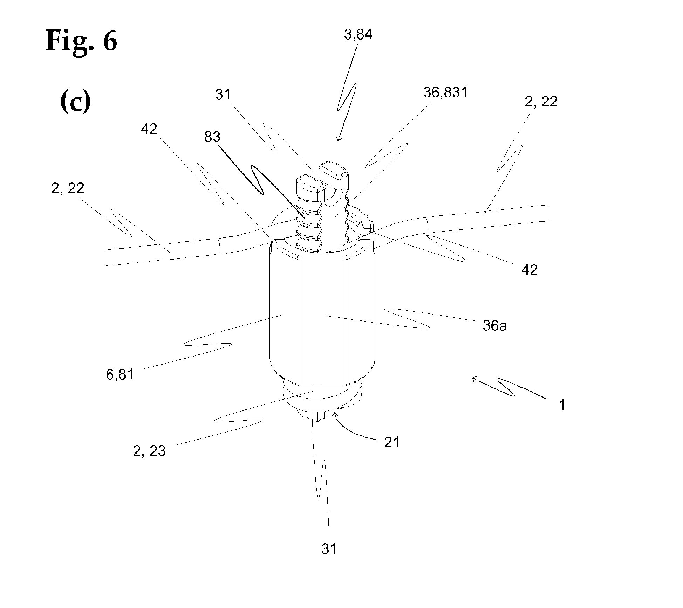

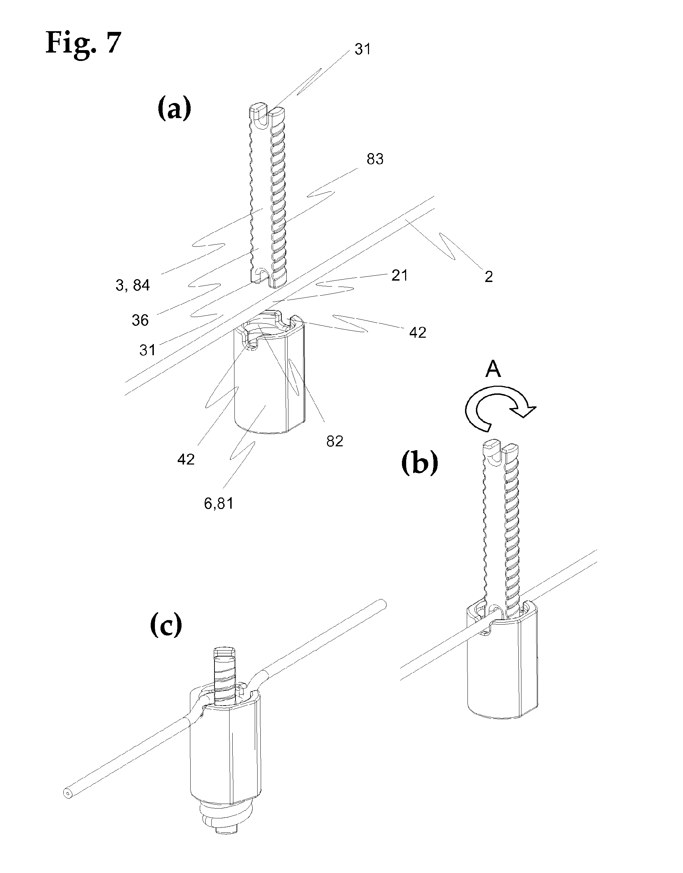

Yet another embodiment of a device according to the invention is shown in Figure 6, (a) and (c) in a perspective view with wound up tubing, and (b) a top view without tubing. In Figure 6(a) a part of the device is shown partially transparent. Figure 7 (a) shows the device (c) before assembly, (b) prior to winding up the tubing, and (c) after winding up the tubing.

The shown embodiment of a device 1 according to the invention comprises a spindle-like spool 3 as an inner unit 84, and a casing 6 as an outer unit 81. In the shown device the inner and outer unit 84, 81 embrace the coiled surplus tubing 23. The inner unit 3, 84 is essentially a cylinder with outer thread 83, of which along two opposite sides the thread is removed 831, resulting in two flat areas 36, where a user can hold the inner unit 84. Both ends of the inner unit 84 are provided with a notch 31 for holding the tubing.

The outer unit 6, 81 is essentially a hollow cylinder, which in the shown embodiment is provided with an inner thread 82 on two opposite sides on the inner surface and two areas 821 without thread. The outer unit 3, 84 further comprises two flat areas 36a on the mantle for better grip. Said inner thread of the outer element is however, although advantageous, not essential for the function of the device.

With the tubing 2 wound up to the device 1, the coiled tubing 23 facing the inner unit 84 interacts with the outer thread 83, and the inner thread 82 interacts with the coiled tubing 23 facing the outer unit 81. Thus the coiled tubing 23 acts as the counter thread for the inner thread 82 of the outer unit 81, and as the counter thread of the outer thread 83 of the inner unit 84. The tubing 2 is wound up as a double helix, a central portion 21 being held in one of the slots 31.

For winding up the tubing 2 on the device 1, the tubing is placed in two opposite notches 42 on the circumference of the outer unit 81, and the notch 31 of the inner unit 84 is also placed on the tubing, both units 81, 84 being aligned (see Figure 7(b)). While holding the outer unit 81, the user rotates the inner unit 84 in a retracting direction A and pushes it toward the outer unit. The notch 31 will force the tubing to rotate, which consequently will form a tubing coil 23 around the outer threading 83. At the same time the notches 42 fixate the tubing in regard to the inner thread 82. Thus by rotating the inner unit 3, 84 the tubing forms a coil 23 that screws itself and the inner unit 84 into the thread 82 of the outer unit 81, at the same time retracting the tubing into the device 1. For releasing the tubing, the inner unit is turned into the opposite direction, hereby reversing the process.

Since both for the inner thread 82 and outer thread 83 the thread is removed on two opposite sides, the tubing coil 23 will be slightly compressed between the threaded portions when a portion of the inner thread 82 passes a portion of the outer thread 83. This results in a resilient force that has to be repeatedly overcome by the user when rotating the inner unit with the coil 23. Thus the inner unit, the tubing coil 23, and the outer unit form a ratchet-like locking mechanism.

A device as shown in Figures 6 and 7 can be attached to already connected tubing and removed without disconnecting the tubing. Due to its oblong form it is space-saving, while at the same time storing a considerably amount of tubing. The device is producible at very low costs, since it consists only of two parts, which have a geometry that is easy to produce, for example with injection moulding.

### List of Reference Numerals

- 1: device
- 2: tubing
- 21: portion of the tubing
- 22, 22': end of infusion tubing
- 3: spool
- 30, 30a: spool compartment
- 31: tube locking structure for positively locking a portion of the tubing
- 311: slot
- 312: slot
- 32: hub
- 33a: upper outer delimiting flange
- 33b: lower outer delimiting flange
- 33c: central delimiting flange
- 34: slot
- 35: bore
- 351: groove
- 36, 36a: grip structure
- 37: spool body
- 4: guiding structure
- 42: opening
- 421: slot
- 5: locking mechanism
- 51: release button
- 52: ratchet mechanism
- 521: paw
- 522: tooth of gear
- 523: hinge area
- 524: ratchet gear
- 525: hinge area
- 526: reverse ramp
- 527: barrier
- 528: neck
- 6: casing
- 61: top cover
- 62: bottom cover
- 63: bridge structure
- 631: pin
- 632: bore
- 64: hub
- 641: slot
- 642: guiding component
- 65: circumferential wall
- 7: actuation mechanism
- 71: spring element, coil spring
- 711: first end of coil spring
- 712: second end of coil spring
- 81: outer unit
- 82: inner threading structure
- 821: area without threading
- 83: outer threading structure
- 831: area without threading
- 84: inner unit
- A: retracting rotation
- B: releasing rotation
- C: path of paw during releasing rotation
- D: path of paw during retracting rotation
- E: path of paw during locking
- F: path of paw during unlocking

## Claims

1. A device (1) for storing and dispensing infusion tubing (2), comprising a casing (6, 81), a spool (3, 84) for storing the tubing (2), rotatably mounted (35, 64) on the casing (6), and a locking structure (31, 311, 312) for positively locking a central portion (21) of a tubing (2) on the spool (3, 84), **characterized by** a ratchet-like locking mechanism (5, 52, 81, 82) suitable for locking a rotation of the spool (3) in regard to the casing (6) in at least one direction (A, B).

2. The device according to claim 1, **characterized in that** the locking-mechanism (5, 52) can lock the rotation of the spool (3) in both directions (A, B).

3. The device according to claim 1 or 2, **characterized by** guiding structures (4, 42) for the tubing (2).

4. The device according to any of claims 1 to 3, **characterized in that** the locking mechanism is a ratchet mechanism (52) comprising a ratchet gear consisting of a multitude of circularly arranged grooves (351) or teeth (522), and one or more paws (521) interacting with the ratchet gear, wherein the ratchet gear is arranged on an outer delimiting flange (33a) of the spool (3), and the paws are attached to the casing (6), or vice versa.

5. The device according to any of claims 1 to 3, **characterized in that** the spool (3) comprises a bore (35) rotatably mounted on a hub (64) attached to the casing (6), and the locking mechanism (5) is a ratchet mechanism (52) comprising a ratchet gear consisting of a multitude of circularly arranged grooves (351) or teeth (522), and one or more paws (521) interacting with the ratchet gear, wherein the ratchet gear is arranged on the circumference of the bore (35), and the paws are arranged on the hub (64), or vice versa.

6. The device according to any of the preceding claims, **characterized by** an actuation mechanism (7), particularly a spring coil (71), for rotating the spool (3) in one direction (A).

7. The device according to any of the preceding claims, **characterized in that** an outer delimiting flange (33a) of the spool (3) comprises two radial slots (312) on opposite sides of the flange (33a), extending to axial slots (312) in the spool body (37).

8. The device according to claim 7, **characterized by** guiding structures (4, 42) for the tubing (2), with openings (42) through which the tubing can run to the spool (3), wherein the openings (42) comprise slots (421) facing toward the same direction as the slots (312).

9. The device according to claim 1, **characterized in that** the spool (3) is an inner unit (84) in the form of a cylinder, having an outer thread (83) and two or more areas (831) without threading; and the casing (6) is an outer unit (81) in the form of a cylinder barrel, wherein the dimensions of the inner (84) and outer (81) unit and the outer thread (83) are chosen in such a way that tubing (2) with a certain diameter can be wound up to the inner unit (84), forming a tubing coil (23), and that said tubing coil can be arranged in the outer unit (81).

10. The device according to claim 9, **characterized in that** the outer unit (81) has an inner thread (82) and two or more areas (821) without threading, wherein the dimensions of the inner (84) and outer (81) unit and the threads (82, 83) are chosen in such a way that tubing (2) with a certain diameter can be wound up to the inner unit (84), forming a tubing coil (23), and that said tubing coil can be screwed into the thread (82) of the outer unit (81).

11. A device (1) according to any of claims 1 to 10, **characterized in that** the device (1) is provided with a tubing (2) wound up to the spool (3).

12. The device according to claim 11, **characterized in that** the device (1) including the tubing (2) is sterilized.

13. The device according to claim 11 or 12, **characterized in that** the tubing (2) comprises connectors on one or both ends (22, 22□) for connecting the tubirto an infusion pump device and/or an infusion cannula or needle.

14. A set comprising a device (1) according to any of claims 1 to 13 and one or more pieces of tubing (2).

15. An infusion tubing (2) with one or more devices (1) according to any of claims 1 to 10 attached to the infusion tubing (2).
